# FASCICULE DE BREVET EUROPEEN

(11) **EP 2 861 205 B1**
(45) Date de publication et mention de la délivrance du brevet: **06.12.2017**
(21) Numéro de dépôt: 13731414.2
(22) Date de dépôt: 12.06.2013
(51) Int. Cl.: A61K 8/49, A61Q 5/00, A61K 8/60, A61Q 7/00

(54) **COMPOSITION COMPRENANT UN ESTER D'ACIDE PYRIDINE DICARBOXYLIQUE ET UN ESTER DE GLUCOSE ET D'ACIDE GRAS.**
ZUSAMMENSETZUNG MIT EINEM PYRIDINDICARBONSÄUREESTER UND GLUCOSEFETTSÄUREESTER.
COMPOSITION INCLUDING A PYRIDINE DICARBOXYLIC ACID ESTER AND A GLUCOSE AND FATTY ACID ESTER.

(30) Priorité: 15.06.2012 FR 1255608; 10.09.2012 US 201261698755 P
(43) Date de publication de la demande: 22.04.2015
(73) Titulaire: L'OREAL, 75008 Paris (FR)
(72) Inventeur: DERKX, Tiphaine, F-75017 Paris (FR); DRILLON, Damien, 93400 SAINT-OUEN (FR); RICHET, Laurence, F-95590 Presles (FR)
(74) Mandataire: Dodin, Catherine
(86) Numéro de dépôt international: PCT/FR2013/051363
(87) Numéro de publication internationale: WO 2013/186485

(56) Documents cités:
- EP-A1- 1 352 629
- EP-A1- 1 371 658
- EP-A1- 1 688 128
- WO-A1-93/02657
- FR-A1- 2 863 876

## Description

La présente invention concerne une composition cosmétique ou pharmaceutique comprenant au moins un dérivé d'acide pyridine-dicarboxylique en association avec au moins un ester de glucose et d'acide(s) gras, ainsi que son utilisation notamment dans le domaine capillaire.

La croissance des cheveux et leur renouvellement sont principalement déterminés par l'activité des follicules pileux et leur environnement matriciel. Leur activité est cyclique et comporte essentiellement trois phases à savoir la phase anagène, la phase catagène et la phase télogène. La chevelure se renouvelle en permanence et sur les 150 000 cheveux environ que comporte une chevelure, 10% environ sont au repos et seront remplacés dans les mois qui viennent.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

Il est connu, par ailleurs, que certains facteurs tels qu'un déséquilibre hormonal, un stress physiologique, la malnutrition, peuvent accentuer le phénomène. En outre, la chute ou l'altération des cheveux peut être en relation avec des phénomènes saisonniers.

Il a ainsi notamment été proposé par le brevet EP1352629, d'utiliser des dérivés d'acide pyridine-dicarboxylique pour induire et/ou stimuler la croissance des fibres kératiniques humaines telles que les cheveux ou les cils, et/ou freiner leur chute et/ou augmenter leur densité.

Toutefois, on a constaté que l'utilisation de tels composés, particulièrement en présence de solvant, pouvait entraîner des sensations d'inconfort du cuir chevelu, telles que des tiraillements, ou encore des problèmes de sécheresse. Dans certains cas, on a également observé des cheveux ternes, sans tonus, ou difficiles à coiffer et à discipliner.

La présente invention a notamment pour but de pallier ces inconvénients en proposant une composition permettant d'obtenir un effet bénéfique le plus étendu possible, ladite composition agissant de manière large sur le cuir chevelu et l'ensemble de la chevelure, du bulbe du cheveu à sa pointe.

Grâce à l'invention, on peut ainsi obtenir d'une part l'effet technique lié à l'utilisation des dérivés d'acide pyridine-dicarboxylique, tout en ayant un bon effet cosmétique sur l'ensemble de la chevelure.

On a constaté que l'association selon l'invention permet d'obtenir, d'une part, une répartition et un étalement de la composition sur la chevelure améliorés, et d'autre part, une amélioration des propriétés de conditionnement des cheveux, notamment en terme de douceur, souplesse, lissage et démêlage.

La présente invention a pour objet une composition comprenant :
- (i) au moins un composé de formule générale (I), ou l'un de ses sels : 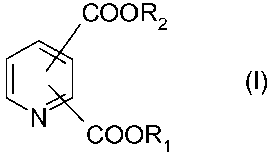 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé; ou un radical aryle en C6-C18;
   lesdits radicaux étant éventuellement substitués par un ou plusieurs groupes choisis parmi OH, NH₂, -OR, -OCOR et -NHR avec R représentant un groupement alkyle en C₁-C₁₈,
   l'un au moins des groupements R1 et R2 étant différent d'un atome d'hydrogène; et
- (ii) au moins un ester de glucose et d'acide(s) gras.

Un autre objet de l'invention concerne l'utilisation d'une composition ainsi définie pour induire et/ou stimuler la croissance des fibres kératiniques humaines et/ou freiner leur chute et/ou augmenter leur densité.

On a constaté que les compositions selon l'invention présentaient de bonnes performances cosmétiques, au niveau de la qualité des cheveux, et également au niveau du confort du cuir chevelu. Le cuir chevelu est apaisé, et aucun tiraillement ou échauffement n'est observé après l'application de la composition. La chevelure est disciplinée, facile à coiffer, les cheveux sont plus forts et plus résistants.

Dans la présente description, l'expression "au moins un" est équivalente à l'expression "un ou plusieurs" et peut y être substituée; et l'expression "compris entre ... et ..." est équivalente à l'expression "allant de ... à ..." et peut y être substituée.

### 1/ Ester d'acide pyridine dicarboxylique

La composition selon l'invention comprend donc au moins un composé qui est un ester d'acide pyridine dicarboxylique. L'ester peut être un monoester ou un diester, de préférence un diester. Il peut également s'agir d'un sel d'un tel ester.

Lesdits composés répondent à la formule générale (I), ou l'un de ses sels : 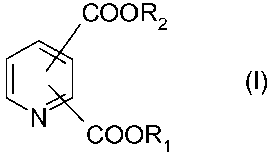 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé (alkyle notamment); ou un radical aryle en C6-C18;
lesdits radicaux hydrocarboné aliphatique ou aryle étant éventuellement substitués par un ou plusieurs groupes choisis parmi OH, NH₂, -OR, -OCOR et -NHR avec R représentant un groupement alkyle en C₁-C₁₈,
l'un au moins des groupements R1 et R2 étant différent d'un atome d'hydrogène.

De préférence, les substituants COOR₁ et COOR₂ sont respectivement en position 2 et 3, ou 2 et 4 du noyau pyridine. Préférentiellement, ils sont en position 2 et 4.

Préférentiellement, les composés répondent à la formule (la), ou un de ses sels : 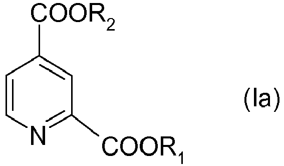 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé (alkyle notamment); ou un radical aryle en C6-C18;
lesdits radicaux hydrocarboné aliphatique ou aryle étant éventuellement substitués par un ou plusieurs groupes choisis parmi OH, NH₂, -OR, -OCOR et -NHR avec R représentant un groupement alkyle en C₁-C₁₈,
l'un au moins des groupements R1 et R2 étant différent d'un atome d'hydrogène.

De préférence, dans les formules (I) et (la), le radical hydrocarboné aliphatique, notamment alkyle, en C₁-C₁₈ est un radical hydrocarboné aliphatique, notamment alkyle, en C1-C10, notamment en C1-C6, tel que méthyle, éthyle, tertiobutyle, isopropyle, hexyle. Ledit radical hydrocarboné aliphatique peut également contenir au moins une double liaison ou une triple liaison carbone-carbone, comme par exemple -CH=CH₂, -CH₂-CH=CH-CH₃ et -CH₂-C≡CH.

Le radical aryle peut représenter le radical phényle ou naphtyle.

En particulier, dans les formules (I) et (Ia), R1 et R2 représentent, indépendamment l'un de l'autre, un atome d'hydrogène, un radical hydrocarboné aliphatique saturé, notamment alkyle, linéaire ou ramifié, en C₁-C₁₈, mieux en C1-C10, voire en C1-C6, éventuellement substitué par un groupe alcoxy ou acyloxy (OR ou OCOR avec R désignant un radical alkyle en C1-C18), l'un au moins des groupements R1 et R2 étant différent d'un atome d'hydrogène.

De préférence, R1 et R2 sont identiques.

Préférentiellement, R₁ et R₂ sont identiques et représentent un radical alkyl saturé et linéaire en C1-C18, de préférence en C1-C10, et encore mieux en C1-C6; et tout particulièrement un radical éthyle.

On peut notamment utiliser les composés de formule (I) suivants :
- le pyridine-2,4-dicarboxylate de diméthyle,
- le pyridine-2,3-dicarboxylate de diméthyle,
- le pyridine-2,4-dicarboxylate de diéthyle,
- le pyridine-2,3-dicarboxylate de diéthyle,
- le pyridine-2,5-dicarboxylate de diéthyle,
- le pyridine-2,5-dicarboxylate de diméthyle,
- le pyridine-2,4-dicarboxylate de diisopropyle,
- le pyridine-2,4-dicarboxylate de di(acétyloxyméthyle) (dérivé de formule (I) tel que R₁ et R₂ représentent -CH₂-O-COCH₃),

Préférentiellement, la composition comprend, comme composé de formule (I) ou (la), le pyridine-2,4-dicarboxylate de diéthyle.

Par sels des composés de formule (I), on entend selon l'invention les sels organiques ou minéraux d'un composé de formule (I), ces sels étant physiologiquement acceptables. Comme sels minéraux, on peut citer les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺); les hydroxydes, les carbonates et les chlorures. Comme sels organiques, on peut citer les sels triéthanolamine, mono-éthanolamine, diéthanolamine, hexadécylamine, N,N,N',N'-tétrakis-(hydroxy-propyl-2) éthylène di-amine et tris-hydroxyméthyl aminométhane.

Les composés de formule (I) sont connus en tant que tels; ils sont notamment décrits, ainsi que leur fabrication, dans le brevet EP1352629.

De préférence, la composition selon l'invention comprend le ou les composés de formule (I) en une quantité comprise entre 0,001 et 10% en poids, notamment de 0,01 à 5% poids, par rapport au poids total de la composition.

### 2/ Ester de glucose et d'acide(s) gras

La composition selon l'invention comprend également au moins un ester de glucose et d'acide(s) gras.

L'ester de glucose et d'acide(s) gras selon l'invention répond à la formule (II) suivante : (RCOO)ₙ-Glu (II)
dans laquelle :
- Glu représente le radical glucose ;
- R représente un radical alkyle ou alkényle en C8-C40 éventuellement hydroxylé ;
- n représente un nombre entier allant de 1 à 5.

Les groupements RCOO sont de préférence issus des acides gras saturés ou insaturés en C8-C32, plus préférentiellement choisis parmi les acides laurique, myristique, palmitique, cétylique, stéarique, oléique, linoléïque, arachidique, béhénique, lauroléïque, myristoléïque, palmitoléïque, linolénique, et leurs mélanges.

Dans une variante préférée de l'invention, l'ester de glucose et d'acide(s) gras est un ester de glucose et de vitamine F.

La vitamine F, composé naturellement présent dans les corps gras et notamment dans l'huile de lin, l'huile de tournesol ou l'huile de carthame, est constituée d'un mélange d'acides gras, principalement en C12-C20. Dans le cadre de la présente invention, on considère que la vitamine F est généralement constituée (% en poids):
- de 70 à 80% en poids d'acide linoléïque, et
- de 10 à 15% en poids d'acide oléïque,
- de 4 à 8% en poids d'acide palmitique,
- de 0,5 à 5% en poids d'acide stéarique, et
- de 0 à 10% en poids d'un ou plusieurs autres acides choisis parmi les acides laurique, myristique, arachidique, béhénique, lauroleïque, myristoleïque, palmitoleïque et linolénique.

Il en résulte que l'ester de vitamine F et de glucose employé dans le cadre de l'invention, qui est susceptible d'être obtenu par estérification, partielle ou totale, de la vitamine F, est de préférence lui-même constitué d'un mélange de différents esters, découlant de la présence des différents acides, formés lors de cette réaction. Par ailleurs, le glucose possédant cinq sites susceptibles de réagir lors de la réaction d'estérification, l'ester de vitamine F et de glucose employé dans le cadre de l'invention peut comprendre également un mélange des esters formés sur les différentes positions du glucose. Enfin, l'ester de vitamine F et de glucose employé dans le cadre de l'invention peut comprendre également un mélange des monoesters et diesters susceptibles d'être formés.

Dans la présente description, on entend par "ester de vitamine F et de glucose", le mélange constitué par l'ensemble des esters, mono ou di, formés lors de la réaction d'estérification, partielle ou totale, de la vitamine F et du glucose.

Ledit ester de vitamine F et de glucose employé dans le cadre de la présente invention comprend donc principalement un mélange de composés qui peuvent être représentés par la formule (IIa) suivante : 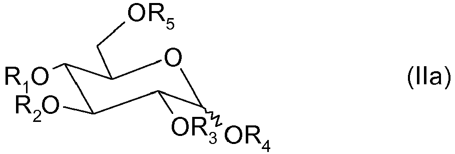 dans laquelle R1, R2, R3, R4 et R5, indépendamment les uns des autres, représentent l'atome d'hydrogène ou un radical -COR' avec R' représentant une chaîne hydrocarbonée aliphatique, saturée ou insaturée (de préférence alkyle), linéaire et comprenant 11 à 21 atomes de carbone, sous réserve qu'au moins un des radicaux R1 à R5 soient différents de l'hydrogène.

Le rapport entre le nombre de fonctions esters du produit estérifié et le nombre de fonctions hydroxyles initiales, ou taux d'estérification, pour une molécule de glucose, est de préférence compris entre 0,2 et 1, notamment de 0,2 à 0,6 et en particulier de 0,21 à 0,4.

Le glucose est estérifié de préférence en position 1, en position 2, en position 3 et/ou en position 6. Préférentiellement, le glucose est estérifié principalement en position 1 et/ou 6.

Le rapport entre le nombre de fonctions esters en position 6 et le nombre total de fonctions esters, pour une molécule de glucose, est de préférence compris entre 0,55 et 1, notamment entre 0,70 et 0,98 et préférentiellement entre 0,90 et 0,97.

Notamment, l'ester de vitamine F et de glucose employé dans le cadre de l'invention peut comprendre au moins un ester, notamment monoester, de glucose et d'acide linoléïque; au moins un ester, notamment monoester, de glucose et d'acide oléïque; au moins un ester, notamment monoester, de glucose et d'acide palmitique; et au moins un ester, notamment monoester, de glucose et d'acide stéarique.

Il peut en outre comprendre au moins un ester, notamment monoester, de glucose et d'un ou plusieurs esters de glucose et d'acide laurique, myristique, arachidique, béhénique, lauroleïque, myristoleïque, palmitoleïque et/ou linolénique.

Il peut en outre également comprendre au moins un diester de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

Ainsi, de préférence, l'ester de vitamine F et de glucose employé dans le cadre de l'invention comprend généralement, toutes positions confondues, :
- de 40 à 80% en poids, de préférence 60 à 75% en poids, préférentiellement 68-72% en poids, d'ester, notamment monoester, de glucose et d'acide linoléïque,
- de 10 à 20% en poids, de préférence 12 à 17% en poids, préférentiellement 14-15% en poids, d'ester, notamment monoester, de glucose et d'acide oléïque,

- de 5 à 20% en poids, de préférence 7 à 15% en poids, préférentiellement 9-12% en poids, d'ester, notamment monoester, de glucose et d'acide palmitique,
- de 0,5 à 7% en poids, de préférence 1 à 5% en poids, préférentiellement 2-4% en poids, d'ester, notamment monoester, de glucose et d'acide stéarique,
- de 0 à 10% en poids, notamment 0,10-4% en poids, voire 0,15-2% en poids, d'un ou plusieurs monoesters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

En particulier, l'ester de vitamine F et de glucose employé dans le cadre de l'invention peut comprendre :
- de 40 à 80% en poids, de préférence 60 à 75% en poids, préférentiellement 68-72% en poids, d'ester de glucose et d'acide linoléïque dont principalement le 6-O-octadeca-9,12-dienoyl-D-glucopyranose, le 1-O-octadeca-9,12-dienoyl-D-glucopyranose, le 2-O-octadeca-9,12-dienoyl-D-glucopyranose et/ou le 3-O-octadeca-9,12-dienoyl-D-glucopyranose,
- de 10 à 20% en poids, de préférence 12 à 17% en poids, préférentiellement 14-15% en poids, d'ester de glucose et d'acide oléïque, dont principalement le 6-O-octadeca-9-enoyl-D-glucopyranose, le 3-0-octadeca-9-enoyl-D-glucopyranose, le 1-0-octadeca-9-enoyl-D-glucopyranose et/ou le 2-O-octadeca-9-enoyl-D-glucopyranose,
- de 5 à 20% en poids, de préférence 7 à 15% en poids, préférentiellement 9-12% en poids, d'ester de glucose et d'acide palmitique, dont principalement le 6-O-hexadecanoyl-D-glucopyranose, le 3-O-hexadecanoyl-D-glucopyranose, le 1-O-hexadecanoyl-D-glucopyranose et/ou le 2-O-hexadecanoyl-D-glucopyranose;
- de 0,5 à 7% en poids, de préférence 1 à 5% en poids, préférentiellement 2-4% en poids, d'ester de glucose et d'acide stéarique, dont principalement le 6-O-octadecanoyl-D-glucopyranose, le 3-O-octadecanoyl-D-glucopyranose, le 1-O-octadecanoyl-D-glucopyranose et/ou le 2-O-octadecanoyl-D-glucopyranose,
- de 0 à 10% en poids, notamment 0,10-4% en poids, voire 0,15-2% en poids, d'un ou plusieurs monoesters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

Les esters de vitamine F et de glucose sont connus en tant que tels; ils sont notamment décrits, ainsi que leur fabrication, dans les brevets EP1837341 et EP1371658.

De préférence, la composition selon l'invention comprend le ou les esters d'acide(s) gras et de glucose en une quantité comprise entre 0,01 et 20% en poids, notamment de 0,05 à 10% en poids, par rapport au poids total de la composition.

### 3. Ingrédients complémentaires

La composition selon l'invention peut être à usage cosmétique ou pharmaceutique. Préférentiellement, la composition selon l'invention est à usage cosmétique. Par "cosmétique", on entend au sens de l'invention une composition d'aspect, d'odeur et de toucher agréables. De préférence, la composition est d'application topique sur la peau et les fibres kératiniques, et plus spécialement sur le cuir chevelu, les cheveux et les cils.

Selon le mode d'application, cette composition peut se présenter sous toutes les formes galéniques normalement utilisées dans le domaine considéré, notamment cosmétique.

Pour une application topique sur la peau, y compris le cuir chevelu, la composition peut avoir la forme notamment d'une solution ou suspension aqueuse, alcoolique ou hydroalcoolique, d'une suspension ou d'une solution huileuse, d'une émulsion ou dispersion de consistance liquide ou semi-liquide obtenue par dispersion d'une phase grasse dans une phase aqueuse (H/E) ou inversement (E/H), d'une dispersion ou émulsion de consistance molle, d'un gel aqueux ou hydroalcoolique ou huileux (anhydre), d'une poudre libre ou compactée à utiliser telle quelle ou à incorporer dans un milieu physiologiquement acceptable (excipient), ou encore de microcapsules ou microparticules, ou de dispersions vésiculaires de type ionique et/ou non ionique.

On peut également envisager une composition sous la forme d'une mousse ou encore sous forme de composition pour aérosol comprenant également un agent propulseur sous pression.

En particulier, la composition selon l'invention peut être une composition capillaire, susceptible d'être appliquée sur le cuir chevelu ou les cheveux, et peut se présenter sous forme d'une lotion de soin capillaire, par exemple d'application journalière ou bihebdomadaire, d'un shampooing ou d'un après-shampooing capillaire, en particulier d'application bihebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Pour une application sur les cils ou les poils, la composition selon l'invention peut se présenter sous forme d'un mascara, pigmenté ou non, à appliquer à la brosse sur les cils ou encore sur les poils de barbe ou de moustache.

Selon un mode de réalisation particulier, la composition selon l'invention se présente sous forme de crème ou lotion capillaire, de shampooing, d'après-shampooing capillaire, ou de mascara capillaire ou pour cils.

Les quantités des différents constituants de la composition selon l'invention sont celles classiquement utilisées dans les domaines considérés. En outre, cette composition est préparée selon les méthodes usuelles. Elle peut ainsi se présenter sous forme d'une lotion, sérum, lait, crème H/E ou E/H, gel, onguent, pommade, poudre, baume, patch, tampon imbibé, savon, pain, mousse.

Lorsque la composition est une émulsion, la proportion de la phase grasse peut aller de 2 à 80% en poids, de préférence de 5 à 50% en poids par rapport au poids total de la composition.

La phase grasse peut contenir des composés gras ou huileux, liquides à température ambiante (25°C) et pression atmosphérique (1 atm), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse est ajustée en fonction de la teneur en phase grasse et en composé(s) de formule (I) ainsi que de celle des éventuels ingrédients additionnels, pour obtenir 100% en poids. En pratique la phase aqueuse représente de préférence 5 à 99,9% en poids.

La phase aqueuse contient de l'eau et éventuellement un solvant organique miscible en toute proportion à l'eau comme les alcools inférieurs en C₁ à C₈ tel que l'éthanol, l'isopropanol, les polyols comme le propylène glycol, le glycérol, le sorbitol ou encore l'acétone.

Les émulsionnants et les coémulsionnants utilisés dans la composition sous forme d'émulsion peuvent être choisis parmi ceux classiquement utilisés dans le domaine cosmétique ou pharmaceutique. L'émulsionnant et/ou le coémulsionnant sont de préférence présents dans la composition en une proportion allant de 0,1 à 30% en poids, de préférence de 0,5 à 20% en poids par rapport au poids total de la composition, mieux de 1 à 8%. Leur nature est, en outre, fonction du sens de l'émulsion. L'émulsion peut, en outre, comprendre des vésicules lipidiques et notamment des liposomes.

Lorsque la composition est une solution ou un gel huileux, la phase grasse peut représenter plus de 90% du poids total de la composition.

Avantageusement, pour une application capillaire, la composition est une solution ou suspension aqueuse, alcoolique ou hydroalcoolique, et mieux une solution ou une suspension eau/éthanol. La fraction alcoolique peut représenter de 5% à 99,9% et mieux de 8% à 80%.

Pour une application mascara, la composition est de préférence une dispersion de cire-dans-eau ou de cire-dans-huile, une huile gélifiée ou un gel aqueux, pigmenté ou non.

Dans un mode de réalisation particulier, la composition selon l'invention peut comprendre au moins une vitamine B et/ou un de ses analogues ou dérivés.

Par vitamine B, on entend au moins l'une des vitamines appartenant à ce groupe, à savoir la vitamine B1 ou thiamine; la vitamine B2 ou riboflavine; la vitamine B3 (ou vitamine PP) ou niacine ou niacinamide; la vitamine B5 ou acide pantothénique; la vitamine B6 ou pyridoxine; la vitamine B8 (ou vitamine H) ou biotine; la vitamine B9 ou acide folique, et la vitamine B12 ou cyanocobalamine.

Comme analogues ou dérivés de vitamine B, on peut notamment citer les sels correspondants, comme le pantothénate de calcium, les pro-vitamines B comme le panthénol, qui est l'alcool analogue de la vitamine B5, encore appelé provitamine B5; on peut également citer les éthers et les esters correspondants, comme le panthényl éthyl éther, le panthényl éthyl éther acétate et le panthényl triacétate. On peut bien évidemment utiliser un mélange de ces différents composés. Préférentiellement, la vitamine B susceptible d'être employée dans le cadre de l'invention est choisie parmi la vitamine B3, la vitamine B5, la vitamine B6 et la vitamine B8, ou l'un de leurs analogues ou dérivés, et notamment le pantothénate de calcium, le panthénol, le panthényl éthyl éther, le panthényl éthyl éther acétate et le panthényl triacétate; ainsi que leurs mélanges.

De préférence, la composition selon l'invention comprend la ou les vitamines B, analogue(s) et/ou dérivé(s) en une quantité comprise entre 0,001 et 5% en poids, notamment 0,005 et 2% en poids, encore mieux entre 0,01 et 1% en poids, par rapport au poids total de la composition.

La composition selon l'invention peut contenir également des adjuvants habituels dans le domaine cosmétique, choisis par exemple parmi les gélifiants ou épaississants hydrophiles ou lipophiles, les additifs hydrophiles ou lipophiles, les conservateurs, les antioxydants (caroténoïdes), les parfums, les charges, les absorbeurs d'odeurs, les électrolytes, les neutralisants, les agents bloqueurs d'U.V. comme les filtres solaires, les polymères filmogènes, les actifs cosmétiques comme les vitamines; les matières colorantes, solubles ou non dans le milieu. Les quantités de ces différents adjuvants sont celles classiquement utilisées dans le domaine cosmétique, par exemple de 0,01 à 20%, mieux de 0,1 à 10% du poids total de la composition. Ces adjuvants, selon leur nature, peuvent être introduits dans la phase grasse, dans la phase aqueuse et/ou dans les vésicules lipidiques et notamment des liposomes.

Comme huiles ou cires susceptibles d'être employées, on peut citer les huiles minérales (huile de vaseline, isoparaffine hydrogénée), les huiles végétales (fraction liquide du beurre de karité, huile de tournesol, de soja, de germes de blé), les huiles animales (perhydrosqualène), les huiles de synthèse (huile de Purcellin, esters d'acide gras), les huiles ou cires siliconées (polydiméthylsiloxanes linéaires ou cycliques, cyclométhicone, phényltriméthicone), les huiles fluorées (perfluoropolyéthers), les cires d'abeille, de Candellila, de riz, de carnauba, de paraffine ou de polyéthylène. On peut également citer des alcools gras et des acides gras (acide stéarique, linoléique, linolénique par exemple).

Comme émulsionnants utilisables dans l'invention, on peut citer par exemple les alcools gras oxyéthylénés, le stéarate ou laurate de glycérol, le stéarate ou oléate de sorbitol polyoxyéthyléné (par exemple le polysorbate 60 et le mélange de PEG-6/PEG-32/Glycol Stéarate vendu sous la dénomination de Tefose® 63 par la société Gattefosse), les (alkyl)diméthicones copolyol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose et leurs mélanges.

La composition peut comprendre d'autres actifs qui peuvent être hydrophiles, comme les protéines ou les hydrolysats de protéine, les acides aminés, les polyols, l'urée, l'allantoïne, les sucres et les dérivés de sucre, les vitamines hydrosolubles, les extraits végétaux, ces extraits pouvant alors contenir ou non des isoflavones; ou qui peuvent être lipophiles, comme le rétinol (vitamine A) et ses dérivés notamment ester (palmitate), le tocophérol (vitamine E) et ses dérivés, notamment ester (acétate palmitate), les acides gras essentiels comme les acides eicosatétraénoïque et eicosatriénoïque ou leurs esters et amides, les céramides, les huiles essentielles, les esters des hydroxy-acides, les phospholipides comme la lécithine; ou qui peuvent être solubles dans des solvants alcooliques comme les lactones (kawaïne); et leurs mélanges.

Elle peut également comprendre des actifs additionnels notamment ceux favorisant la repousse des fibres kératiniques humaines et/ou limitant leur chute. On peut notamment citer, seul ou en mélange :
- les agents antibactériens; les antiparasitaires, les antifongiques;
- les extraits de micro-organismes notamment les extraits bactériens ;
- les agents modulant la différenciation et/ou la prolifération cutanée tels que l'acide rétinoïque et ses isomères, le rétinol et ses esters, la vitamine D et ses dérivés, les oestrogènes tels que l'oestradiol ;
- les agents modulant l'adhésion bactérienne sur la peau et /ou les muqueuses tels que le miel, notamment le miel d'acacias et certains dérivés de sucres ;
- l'acide acétylsalicylique, l'acétaminophène ou l'acide glycyrrhétinique, l'α-bisabolol;
- les agents kératolytiques tels que les acides alpha- et bêta-hydroxycarboxyliques ou bêta-cétocarboxyliques, leurs sels, amides ou esters, les lactones et leurs sels correspondants, et plus particulièrement les hydroxyacides tels que l'acide glycolique, l'acide lactique, l'acide salicylique, l'acide citrique et de manière générale les acides de fruits, les dérivés de l'acide salicylique tels que ceux porteurs d'un radical alcanoyle ayant de 2 à 12 atomes de carbone en position 5 du cycle benzénique comme l'acide n-octanoyl-5-salicylique ;
- les agents anti-radicaux libres tels que l'alpha-tocophérol ou ses esters, les superoxyde dismutases, certains chélatants de métaux ou l'acide ascorbique et ses esters ;
- les anti-séborrhéiques;
- les antipelliculaires comme l'octopirox ou la pyrithione de zinc ;
- les agents diminuant la chute des cheveux comme l'aminexil, le 6-O-[(9Z,12Z)-octadéca-9,12-diènoyl]hexapyranose; les dérivés de pyrimidine, comme le 2,4-diamino 6-pipéridinopyrimidine 3-oxyde ou "minoxidil" décrit dans les brevets US4139619 et US4596812.

La composition selon l'invention peut en outre comprendre des substances telles que les antagonistes de substance P, de CGRP ou de bradykinine et les inhibiteurs de NO-synthase, composés décrits comme étant actifs dans le traitement des peaux sensibles et comme présentant des effets anti-irritants, en particulier vis-à-vis de composés irritants éventuellement présents dans les compositions.

La composition selon l'invention, en particulier cosmétique, peut être appliquée sur les zones alopéciques du cuir chevelu et des cheveux, éventuellement laissée en contact plusieurs heures et/ou éventuellement rincée.

On peut, par exemple, appliquer la composition selon l'invention le soir, garder celle-ci en contact toute la nuit et éventuellement effectuer un shampooing le matin. Ces applications peuvent être renouvelées quotidiennement pendant un ou plusieurs mois suivant les individus.

La présente invention a également pour objet un procédé de traitement cosmétique des matières kératiniques humaines, notamment des cheveux et/ou de la peau, y compris le cuir chevelu, dans lequel on applique sur lesdites matières kératiniques une composition cosmétique telle que définie ci-dessus, et éventuellement à rincer lesdites matières kératiniques.

Plus spécialement, ledit procédé est un procédé de soin cosmétique des cheveux et/ou du cuir chevelu, en vue d'améliorer leur état et/ou leur aspect.

Ce procédé de traitement présente bien les caractéristiques d'un procédé cosmétique dans la mesure où il permet d'améliorer l'esthétique des matières, notamment des fibres, kératiniques humaines en leur donnant une plus grande vigueur, un aspect amélioré (apport de conditionnement), une plus grande facilité de manipulation (étalement, répartition) et de coiffage.

L'invention est décrite plus en détail dans les exemples suivants.

### Exemple 1

On prépare la composition suivante (% en poids):
- diéthyl ester d'acide pyridine-2,4-dicarboxylique
   (nom INCI : DIETHYLLUTIDINATE) 5%
- éthanol 56,5%
- ester de vitamine F et de glucose (exemple 1 de EP1371658) protégé par 0,5% de tocophérols et 0,1% de citrate trisodique (nom INCI : Safflower glucoside) 0,1%
- acide citrique qs pH = 5,5
- eau qsp 100%

Cette lotion est limpide et stable dans le temps (au moins 2 mois, à 25°C ou 45°C).

Appliquée raie par raie sur le cuir chevelu, cette lotion présente de bonnes qualités d'usage en favorisant la distribution et la répartition des actifs sur le cuir chevelu. Elle présente également de bonnes performances cosmétiques. Les cheveux sont soyeux et légers, la chevelure est disciplinée et facile à coiffer.

## Revendications

1. Composition comprenant :
- (i) au moins un composé de formule générale (I), ou l'un de ses sels : 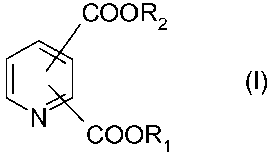 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé; ou un radical aryle en C6-C18;
lesdits radicaux étant éventuellement substitués par un ou plusieurs groupes choisis parmi OH, NH₂, -OR, -OCOR et -NHR avec R représentant un groupement alkyle en C₁-C₁₈,
l'un au moins des groupements R1 et R2 étant différent d'un atome d'hydrogène; et
- (ii) au moins un ester de glucose et d'acide(s) gras.

2. Composition selon la revendication 1, dans laquelle les substituants COOR₁ et COOR₂ sont respectivement en position 2 et 3, ou 2 et 4 du noyau pyridine; préférentiellement en position 2 et 4.

3. Composition selon l'une des revendications précédentes, dans laquelle R1 et R2 sont identiques.

4. Composition selon l'une des revendications précédentes, dans laquelle R1 et R2 représentent, indépendamment l'un de l'autre, un atome d'hydrogène, un radical hydrocarboné aliphatique saturé, notamment alkyle, linéaire ou ramifié, en C₁-C₁₈, mieux en C1-C10, voire en C1-C6, éventuellement substitué par un groupe alcoxy ou acyloxy (OR ou OCOR avec R désignant un radical alkyle en C1-C18), l'un au moins des groupements R1 et R2 étant différent d'un atome d'hydrogène.

5. Composition selon l'une des revendications précédentes, dans laquelle R₁ et R₂ sont identiques et représentent un radical alkyl saturé et linéaire en C1-C18, de préférence en C1-C10, et encore mieux en C1-C6; et tout particulièrement un radical éthyle.

6. Composition selon l'une des revendications précédentes, comprenant comme composé de formule (I), l'un des composés suivants :
- le pyridine-2,4-dicarboxylate de diméthyle,
- le pyridine-2,3-dicarboxylate de diméthyle,
- le pyridine-2,4-dicarboxylate de diéthyle,
- le pyridine-2,3-dicarboxylate de diéthyle,
- le pyridine-2,5-dicarboxylate de diéthyle,
- le pyridine-2,5-dicarboxylate de diméthyle,
- le pyridine-2,4-dicarboxylate de diisopropyle,
- le pyridine-2,4-dicarboxylate de di(acétyloxyméthyle) (dérivé de formule (I) tel que R₁ et R₂ représentent -CH₂-O-COCH₃),

7. Composition selon l'une des revendications précédentes, dans laquelle le ou les composés de formule (I) sont présents en une quantité comprise entre 0,001 et 10% en poids, notamment de 0,01 à 5% poids, par rapport au poids total de la composition.

8. Composition selon l'une des revendications précédentes, dans laquelle l'ester de glucose et d'acide(s) gras répond à la formule (II) suivante : (RCOO)ₙ-Glu (II) dans laquelle :
- Glu représente le radical glucose ;
- R représente un radical alkyle ou alkényle en C8-C40 éventuellement hydroxylé ;
- n représente un nombre entier allant de 1 à 5.

9. Composition selon l'une des revendications précédentes, dans laquelle, dans la formule (II), les groupements RCOO sont issus des acides gras saturés ou insaturés en C8-C32, plus préférentiellement choisis parmi les acides laurique, myristique, palmitique, cétylique, stéarique, oléique, linoléïque, arachidique, béhénique, lauroléïque, myristoléïque, palmitoléïque, linolénique, et leurs mélanges.

10. Composition selon l'une des revendications précédentes, dans laquelle l'ester de glucose et d'acide(s) gras est un ester de glucose et de vitamine F.

11. Composition selon l'une des revendications précédentes, dans laquelle l'ester de glucose et d'acide(s) gras comprend un mélange de composés de formule (IIa): 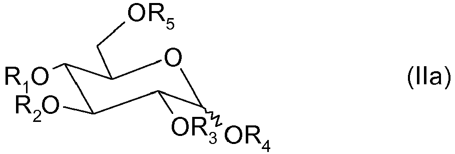 dans laquelle R1, R2, R3, R4 et R5, indépendamment les uns des autres, représentent l'atome d'hydrogène ou un radical -COR' avec R' représentant une chaîne hydrocarbonée aliphatique, saturée ou insaturée (de préférence alkyle), linéaire et comprenant 11 à 21 atomes de carbone, sous réserve qu'au moins un des radicaux R1 à R5 soient différents de l'hydrogène.

12. Composition selon l'une des revendications 10 à 11, dans laquelle l'ester de vitamine F et de glucose comprend, toutes positions confondues, :
- de 40 à 80% en poids, de préférence 60 à 75% en poids, préférentiellement 68-72% en poids, d'ester, notamment monoester, de glucose et d'acide linoléïque,
- de 10 à 20% en poids, de préférence 12 à 17% en poids, préférentiellement 14-15% en poids, d'ester, notamment monoester, de glucose et d'acide oléïque,
- de 5 à 20% en poids, de préférence 7 à 15% en poids, préférentiellement 9-12% en poids, d'ester, notamment monoester, de glucose et d'acide palmitique,
- de 0,5 à 7% en poids, de préférence 1 à 5% en poids, préférentiellement 2-4% en poids, d'ester, notamment monoester, de glucose et d'acide stéarique,
- de 0 à 10% en poids, notamment 0,10-4% en poids, voire 0,15-2% en poids, d'un ou plusieurs monoesters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

13. Composition selon l'une des revendications 10 à 12, dans laquelle l'ester de vitamine F et de glucose comprend :
- de 40 à 80% en poids, de préférence 60 à 75% en poids, préférentiellement 68-72% en poids, d'ester de glucose et d'acide linoléïque dont principalement le 6-O-octadeca-9,12-dienoyl-D-glucopyranose, le 1-O-octadeca-9,12-dienoyl-D-glucopyranose, le 2-O-octadeca-9,12-dienoyl-D-glucopyranose et/ou le 3-O-octadeca-9,12-dienoyl-D-glucopyranose,
- de 10 à 20% en poids, de préférence 12 à 17% en poids, préférentiellement 14-15% en poids, d'ester de glucose et d'acide oléïque, dont principalement le 6-O-octadeca-9-enoyl-D-glucopyranose, le 3-O-octadeca-9-enoyl-D-glucopyranose, le 1-0-octadeca-9-enoyl-D-glucopyranose et/ou le 2-O-octadeca-9-enoyl-D-glucopyranose,
- de 5 à 20% en poids, de préférence 7 à 15% en poids, préférentiellement 9-12% en poids, d'ester de glucose et d'acide palmitique, dont principalement le 6-O-hexadecanoyl-D-glucopyranose, le 3-O-hexadecanoyl-D-glucopyranose, le 1-O-hexadecanoyl-D-glucopyranose et/ou le 2-O-hexadecanoyl-D-glucopyranose;
- de 0,5 à 7% en poids, de préférence 1 à 5% en poids, préférentiellement 2-4% en poids, d'ester de glucose et d'acide stéarique, dont principalement le 6-O-octadecanoyl-D-glucopyranose, le 3-O-octadecanoyl-D-glucopyranose, le 1-O-octadecanoyl-D-glucopyranose et/ou le 2-O-octadecanoyl-D-glucopyranose,
- de 0 à 10% en poids, notamment 0,10-4% en poids, voire 0,15-2% en poids, d'un ou plusieurs monoesters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

14. Composition selon l'une des revendications précédentes, dans laquelle le ou les esters d'acide(s) gras et de glucose sont présents en une quantité comprise entre 0,01 et 20% en poids, notamment de 0,05 à 10% en poids, par rapport au poids total de la composition.

15. Composition selon l'une des revendications précédentes, se présentant sous forme d'une composition cosmétique capillaire, telle qu'une lotion de soin capillaire, d'un shampooing ou d'un après-shampooing capillaire, d'un savon liquide ou solide de nettoyage du cuir chevelu, d'un produit de mise en forme de la coiffure, d'un masque traitant, d'une crème ou d'un gel moussant de nettoyage des cheveux.

16. Procédé de traitement cosmétique (non-thérapeutique) des matières kératiniques humaines, notamment des cheveux et/ou du cuir chevelu, dans lequel on applique sur lesdites matières kératiniques une composition cosmétique telle que définie à l'une des revendications 1 à 15, et éventuellement à rincer lesdites matières kératiniques.

17. Procédé selon la revendication 16, ledit procédé étant un procédé de soin cosmétique des cheveux et/ou du cuir chevelu, en vue d'améliorer leur état et/ou leur aspect.

18. Utilisation non-thérapeutique d'une composition telle que définie à l'une des revendications 1 à 15 pour induire et/ou stimuler la croissance des fibres kératiniques humaines et/ou freiner leur chute et/ou augmenter leur densité.

## Patentansprüche

1. Zusammensetzung, umfassend:
- (i) mindestens eine Verbindung der allgemeinen Formel (I) oder eines ihrer Salze: 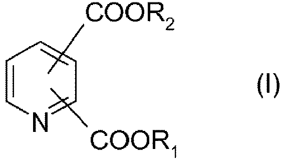 wobei R₁ und R₂ unabhängig voneinander für ein Wasserstoffatom, einen gesättigten oder ungesättigten, linearen oder verzweigten aliphatischen C₁-C₁₈-Kohlenwasserstoffrest oder einen C₆-C₁₈-Arylrest stehen;
wobei die Reste gegebenenfalls durch eine oder mehrere Gruppen, die aus OH, NH₂, -OR, -OCOR und -NHR ausgewählt sind, substituiert sind, wobei R für eine C₁-C₁₈-Alkylgruppe steht,
wobei mindestens eine der Gruppen R₁ und R₂ von einem Wasserstoffatom verschieden ist;
und
- (ii) mindestens einen Ester von Glucose und Fettsäure(n).

2. Zusammensetzung nach Anspruch 1, wobei die Substituenten COOR₁ und COOR₂ respektive in der 2-und 3- oder 2- und 4-Position des Pyridinkerns, vorzugsweise in der 2- und 4-Position, stehen.

3. Zusammensetzung nach einem der vorhergehenden Ansprüche, wobei R₁ und R₂ gleich sind.

4. Zusammensetzung nach einem der vorhergehenden Ansprüche, wobei R₁ und R₂ unabhängig voneinander für ein Wasserstoffatom oder einen gesättigten, linearen oder verzweigten aliphatischen C₁-C₁₈-, besser C₁-C₁₀- oder sogar C₁-C₆-Kohlenwasserstoffrest, insbesondere -Alkylrest, der gegebenenfalls durch eine Alkoxy- oder Acyloxygruppe (OR oder OCOR, wobei R für einen C₁-C₁₈-Alkylrest steht) substituiert ist, stehen, wobei mindestens eine der Gruppen R₁ und R₂ von einem Wasserstoffatom verschieden ist.

5. Zusammensetzung nach einem der vorhergehenden Ansprüche, wobei R₁ und R₂ gleich sind und für einen gesättigten linearen C₁-C₁₈-, vorzugsweise C₁-C₁₀- und noch besser C₁-C₆-Alkylrest und ganz besonders einen Ethylrest stehen.

6. Zusammensetzung nach einem der vorhergehenden Ansprüche, die als Verbindung der Formel (I) eine der folgenden Verbindungen umfasst:
- Pyridin-2,4-dicarbonsäuredimethylester,
- Pyridin-2,3-dicarbonsäuredimethylester,
- Pyridin-2,4-dicarbonsäurediethylester,
- Pyridin-2,3-dicarbonsäurediethylester,
- Pyridin-2,5-dicarbonsäurediethylester,
- Pyridin-2,5-dicarbonsäuredimethylester,
- Pyridin-2,4-dicarbonsäurediisopropylester,
- Pyridin-2,4-dicarbonsäuredi(acetyloxymethyl)-ester (Derivat der Formel (I), das so beschaffen ist, dass R₁ und R₂ für -CH₂-O-COCH₃ stehen).

7. Zusammensetzung nach einem der vorhergehenden Ansprüche, wobei die Verbindung bzw. die Verbindungen der Formel (I) in einer Menge zwischen 0,001 und 10 Gew.-%, insbesondere von 0,01 bis 5 Gew.-%, bezogen auf das Gesamtgewicht der Zusammensetzung, vorliegt bzw. vorliegen.

8. Zusammensetzung nach einem der vorhergehenden Ansprüche, wobei der Ester von Glucose und Fettsäure(n) der folgenden Formel (II) entspricht:
(RCOO)ₙ-Glu (II)
wobei:
- Glu für den Glucoserest steht;
- R für einen gegebenenfalls hydroxylierten C₈-C₄₀-Alkyl- oder -Alkenylrest steht;
- n für eine ganze Zahl im Bereich von 1 bis 5 steht.

9. Zusammensetzung nach einem der vorhergehenden Ansprüche, wobei in der Formel (II) die RCOO-Gruppen von gesättigten oder ungesättigten C₈-C₃₂-Fettsäuren, die weiter bevorzugt aus Laurinsäure, Myristinsäure, Palmitinsäure, Cetylsäure, Stearinsäure, Ölsäure, Linolsäure, Arachinsäure, Behensäure, Lauroleinsäure, Myristoleinsäure, Palmitoleinsäure, Linolensäure und Mischungen davon ausgewählt sind, abstammt.

10. Zusammensetzung nach einem der vorhergehenden Ansprüche, wobei es sich bei dem Ester von Glucose und Fettsäure(n) um einen Ester von Glucose und Vitamin F handelt.

11. Zusammensetzung nach einem der vorhergehenden Ansprüche, wobei der Ester von Glucose und Fettsäure(n) eine Mischung von Verbindungen der Formel (IIa): 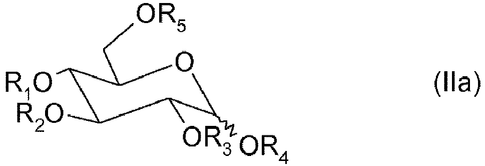 umfasst, wobei R₁, R₂, R₃, R₄ und R₅ unabhängig voneinander für das Wasserstoffatom oder einen Rest -COR' stehen, wobei R' für eine gesättigte oder ungesättigte lineare aliphatische Kohlenwasserstoffkette (vorzugsweise Alkylkette) mit 11 bis 21 Kohlenstoffatomen steht, mit der Maßgabe, dass mindestens einer der Reste R₁ bis R₅ von Wasserstoff verschieden ist.

12. Zusammensetzung nach einem der Ansprüche 10 bis 11, wobei der Ester von Vitamin F und Glucose bei Zusammennahme aller Positionen Folgendes umfasst:
- 40 bis 80 Gew.-%, vorzugsweise 60 bis 75 Gew.-%, bevorzugt 68-72 Gew.-%, Ester, insbesondere Monoester, von Glucose und Linolsäure,
- 10 bis 20 Gew.-%, vorzugsweise 12 bis 70 Gew.-%, bevorzugt 14-15 Gew.-%, Ester, insbesondere Monoester, von Glucose und Ölsäure,
- 5 bis 20 Gew.-%, vorzugsweise 7 bis 15 Gew.-%, bevorzugt 9-12 Gew.-%, Ester, insbesondere Monoester, von Glucose und Palmitinsäure,
- 0,5 bis 7 Gew.-%, vorzugsweise 1 bis 5 Gew.-%, bevorzugt 2-4 Gew.-%, Ester, insbesondere Monoester, von Glucose und Stearinsäure,
- 0 bis 10 Gew.-%, insbesondere 0,10-4 Gew.-% oder sogar 0,15-2 Gew.-% eines oder mehrerer Monoester von Glucose und Laurinsäure, Myristinsäure, Arachinsäure, Behensäure, Lauroleinsäure, Myristoleinsäure, Palmitoleinsäure und/oder Linolensäure,
- 0 bis 10 Gew.-%, insbesondere 0,10-4 Gew.-% oder sogar 0,15-2 Gew.-% Diester von Glucose und einer oder mehreren Säuren, die aus Laurinsäure, Myristinsäure, Arachinsäure, Behensäure, Lauroleinsäure, Myristoleinsäure, Palmitoleinsäure, Linolensäure, Ölsäure, Palmitinsäure, Stearinsäure und/oder Linolensäure ausgewählt sind.

13. Zusammensetzung nach einem der Ansprüche 10 bis 12, wobei der Ester von Vitamin F und Glucose Folgendes umfasst:
- 40 bis 80 Gew.-%, vorzugsweise 60 bis 75 Gew.-%, bevorzugt 68-72 Gew.-%, Ester von Glucose und Linolsäure, darunter hauptsächlich 6-O-Octadeca-9,12-dienoyl-D-glucopyranose, 1-O-Octadeca-9,12-dienoyl-D-glucopyranose, 2-O-Octadeca-9,12-dienoyl-D-glucopyranose und/oder 3-O-Octadeca-9,12-dienoyl-D-glucopyranose,
- 10 bis 20 Gew.-%, vorzugsweise 12 bis 17 Gew.-%, bevorzugt 14-15 Gew.-%, Ester von Glucose und Ölsäure, darunter hauptsächlich 6-O-Octadeca-9-enoyl-D-glucopyranose, 3-O-Octadeca-9-enoyl-D-glucopyranose, 1-O-Octadeca-9-enoyl-D-glucopyranose und/oder 2-O-Octadeca-9-enoyl-D-glucopyranose,
- 5 bis 20 Gew.-%, vorzugsweise 7 bis 15 Gew.-%, bevorzugt 9-12 Gew.-%, Ester von Glucose und Palmitinsäure, darunter hauptsächlich 6-O-Hexadecanoyl-D-glucopyranose, 3-O-Hexadecanoyl-D-glucopyranose, 1-O-Hexadecanoyl-D-glucopyranose und/oder 2-O-Hexadecanoyl-D-glucopyranose,
- 0,5 bis 7 Gew.-%, vorzugsweise 1 bis 5 Gew.-%, bevorzugt 2-4 Gew.-%, Ester von Glucose und Stearinsäure, darunter hauptsächlich 6-O-Octadecanoyl-D-glucopyranose, 3-O-Octadecanoyl-D-glucopyranose, 1-O-Octadecanoyl-D-glucopyranose und/oder 2-O-Octadecanoyl-D-glucopyranose,
- 0 bis 10 Gew.-%, insbesondere 0,10-4 Gew.-% oder sogar 0,15-2 Gew.-% eines oder mehrerer Monoester von Glucose und Laurinsäure, Myristinsäure, Arachinsäure, Behensäure, Lauroleinsäure, Myristoleinsäure, Palmitoleinsäure und/oder Linolensäure,
- 0 bis 10 Gew.-%, insbesondere 0,10-4 Gew.-% oder sogar 0,15-2 Gew.-% Diester von Glucose und einer oder mehreren Säuren, die aus Laurinsäure, Myristinsäure, Arachinsäure, Behensäure, Lauroleinsäure, Myristoleinsäure, Palmitoleinsäure, Linolsäure, Ölsäure, Palmitinsäure, Stearinsäure und/oder Linolensäure ausgewählt sind.

14. Zusammensetzung nach einem der vorhergehenden Ansprüche, wobei der Ester bzw. die Ester von Fettsäure(n) und Glucose in einer Menge zwischen 0,01 und 20 Gew.-%, insbesondere von 0,05 bis 10 Gew.-%, bezogen auf das Gesamtgewicht der Zusammensetzung, vorliegt bzw. vorliegen.

15. Zusammensetzung nach einem der vorhergehenden Ansprüche in Form einer haarkosmetischen Zusammensetzung, wie einer Haarpflegelotion, eines Haarshampoos oder Haarconditioners, einer flüssigen oder festen Seife zur Reinigung der Kopfhaut, eines Produkts zur Gestaltung der Frisur, einer Behandlungsmaske, einer Creme oder eines schäumenden Gels zur Reinigung der Haare.

16. Verfahren zur (nichttherapeutischen) kosmetischen Behandlung von menschlichen Keratinmaterialien, insbesondere den Haaren und/oder oder der Kopfhaut, bei dem man auf die Keratinmaterialien eine kosmetische Zusammensetzung gemäß einem der Ansprüche 1 bis 15 aufbringt und die Keratinmaterialien gegebenenfalls spült.

17. Verfahren nach Anspruch 16, wobei es sich bei dem Verfahren um ein Verfahren zur kosmetischen Pflege der Haare und/oder der Kopfhaut zur Verbesserung ihres Zustands und/oder ihres Aussehens handelt.

18. Nichttherapeutische Verwendung einer Zusammensetzung gemäß einem der Ansprüche 1 bis 15 zur Induzierung und/oder Stimulierung des Wachstums von menschlichen Keratinfasern und/oder zur Verlangsamung ihres Ausfallens und/oder zur Erhöhung ihrer Dichte.

## Claims

1. Composition comprising:
- (i) at least one compound of general formula (I), or one of its salts: 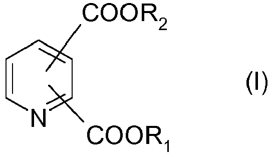 in which R₁ and R₂ represent, independently of one another, a hydrogen atom, a saturated or unsaturated and linear or branched aliphatic C₁-C₁₈ hydrocarbon radical or a C₆-C₁₈ aryl radical;
said radicals optionally being substituted by one or more groups chosen from OH, NH₂, -OR, -OCOR and -NHR with R representing a C₁-C₁₈ alkyl group,
at least one of the R₁ and R₂ groups being other than a hydrogen atom;
and
- (ii) at least one ester of glucose and of fatty acid(s).

2. Composition according to Claim 1, in which the COOR₁ and COOR₂ substituents are respectively in the 2 and 3, or 2 and 4, positions of the pyridine nucleus, preferably in the 2 and 4 positions.

3. Composition according to either of the preceding claims, in which R₁ and R₂ are identical.

4. Composition according to one of the preceding claims, in which R₁ and R₂ represent, independently of one another, a hydrogen atom or a saturated, linear or branched, aliphatic C₁-C₁₈, better still C₁-C₁₀, indeed even C₁-C₆, hydrocarbon radical, in particular alkyl radical, which is optionally substituted by an alkoxy or acyloxy group (OR or OCOR with R denoting a C₁-C₁₈ alkyl radical), at least one of the R₁ and R₂ groups being other than a hydrogen atom.

5. Composition according to one of the preceding claims, in which R₁ and R₂ are identical and represent a saturated linear C₁-C₁₈, preferably C₁-C₁₀ and better still C₁-C₆ alkyl radical and very particularly an ethyl radical.

6. Composition according to one of the preceding claims, comprising, as compound of formula (I), one of the following compounds:
- dimethyl pyridine-2,4-dicarboxylate,
- dimethyl pyridine-2,3-dicarboxylate,
- diethyl pyridine-2,4-dicarboxylate,
- diethyl pyridine-2,3-dicarboxylate,
- diethyl pyridine-2,5-dicarboxylate,
- dimethyl pyridine-2,5-dicarboxylate,
- diisopropyl pyridine-2,4-dicarboxylate,
- di(acetyloxymethyl) pyridine-2,4-dicarboxylate (derivative of formula (I) such that R₁ and R₂ represent -CH₂-O-COCH₃).

7. Composition according to one of the preceding claims, in which the compound or compounds of formula (I) are present in an amount of between 0.001% and 10% by weight, in particular from 0.01% to 5% by weight, with respect to the total weight of the composition.

8. Composition according to one of the preceding claims, in which the ester of glucose and of fatty acid(s) corresponds to the following formula (II):
(RCOO)ₙ-Glu (II)
in which:
- Glu represents the glucose radical;
- R represents an optionally hydroxylated C8-C40 alkyl or alkenyl radical;
- n represents an integer ranging from 1 to 5.

9. Composition according to one of the preceding claims, in which, in the formula (II), the RCOO groups result from saturated or unsaturated C8-C32 fatty acids more preferably chosen from lauric acid, myristic acid, palmitic acid, cetylic acid, stearic acid, oleic acid, linoleic acid, arachidic acid, behenic acid, lauroleic acid, myristoleic acid, palmitoleic acid or linolenic acid and their mixtures.

10. Composition according to one of the preceding claims, in which the ester of glucose and of fatty acid(s) is an ester of glucose and of vitamin F.

11. Composition according to one of the preceding claims, in which the ester of glucose and of fatty acid(s) comprises a mixture of compounds of formula (IIa): 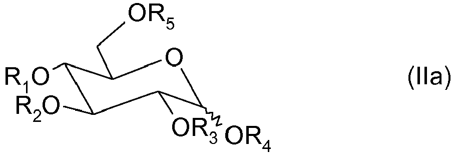 in which R1, R2, R3, R4 and R5 represent, independently of one another, the hydrogen atom or a -COR' radical with R' representing a saturated or unsaturated (preferably alkyl) and linear aliphatic hydrocarbon chain comprising from 11 to 21 carbon atoms, with the proviso that at least one of the R1 to R5 radicals is other than hydrogen.

12. Composition according to either of Claims 10 and 11, in which the ester of vitamin F and of glucose comprises, all positions taken into account:
- from 40% to 80% by weight, preferably from 60% to 75% by weight, preferentially from 68% to 72% by weight, of ester, in particular monoester, of glucose and of linoleic acid,
- from 10% to 20% by weight, preferably from 12% to 17% by weight, preferentially from 14% to 15% by weight, of ester, in particular monoester, of glucose and of oleic acid,
- from 5% to 20% by weight, preferably from 7% to 15% by weight, preferentially from 9% to 12% by weight, of ester, in particular monoester, of glucose and of palmitic acid,
- from 0.5% to 7% by weight, preferably from 1% to 5% by weight, preferentially from 2% to 4% by weight, of ester, in particular monoester, of glucose and of stearic acid,
- from 0% to 10% by weight, in particular from 0.10% to 4% by weight, indeed even from 0.15% to 2% by weight, of one or more monoesters of glucose and of lauric acid, myristic acid, arachidic acid, behenic acid, lauroleic acid, myristoleic acid, palmitoleic acid and/or linolenic acid,
- from 0% to 10% by weight, in particular from 0.10% to 4% by weight, indeed even from 0.15% to 2% by weight, of diesters of glucose and of one or more acids chosen from lauric acid, myristic acid, arachidic acid, behenic acid, lauroleic acid, myristoleic acid, palmitoleic acid, linoleic acid, oleic acid, palmitic acid, stearic acid and/or linolenic acid.

13. Composition according to one of Claims 10 to 12, in which the ester of vitamin F and of glucose comprises:
- from 40% to 80% by weight, preferably from 60% to 75% by weight, preferentially from 68% to 72% by weight, of ester of glucose and of linoleic acid, including mainly 6-O-octadeca-9,12-dienoyl-D-glucopyranose, 1-O-octadeca-9,12-dienoyl-D-glucopyranose, 2-0-octadeca-9,12-dienoyl-D-glucopyranose and/or 3-0-octadeca-9,12-dienoyl-D-glucopyranose,
- from 10% to 20% by weight, preferably from 12% to 17% by weight, preferentially from 14% to 15% by weight, of ester of glucose and of oleic acid, including mainly 6-O-octadeca-9-enoyl-D-glucopyranose, 3-0-octadeca-9-enoyl-D-glucopyranose, 1-0-octadeca-9-enoyl-D-glucopyranose and/or 2-0-octadeca-9-enoyl-D-glucopy-ranose,
- from 5% to 20% by weight, preferably from 7% to 15% by weight, preferentially from 9% to 12% by weight, of ester of glucose and of palmitic acid, including mainly 6-O-hexadecanoyl-D-glucopyranose, 3-0-hexadecanoyl-D-glucopyranose, 1-0-hexadecanoyl-D-glucopyranose and/or 2-O-hexadecanoyl-D-glucopyranose,
- from 0.5% to 7% by weight, preferably from 1% to 5% by weight, preferentially from 2% to 4% by weight, of ester of glucose and of stearic acid, including mainly 6-O-octadecanoyl-D-glucopyranose, 3-0-octadecanoyl-D-glucopyranose, 1-0-octadecanoyl-D-glucopyranose and/or 2-O-octadecanoyl-D-glucopyranose,
- from 0% to 10% by weight, in particular from 0.10% to 4% by weight, indeed even from 0.15% to 2% by weight, of one or more monoesters of glucose and of lauric acid, myristic acid, arachidic acid, behenic acid, lauroleic acid, myristoleic acid, palmitoleic acid and/or linolenic acid,
- from 0% to 10% by weight, in particular from 0.10% to 4% by weight, indeed even from 0.15% to 2% by weight, of diesters of glucose and of one or more acids chosen from lauric acid, myristic acid, arachidic acid, behenic acid, lauroleic acid, myristoleic acid, palmitoleic acid, linoleic acid, oleic acid, palmitic acid, stearic acid and/or linolenic acid.

14. Composition according to one of the preceding claims, in which the ester or esters of fatty acid(s) and of glucose are present in an amount of between 0.01% and 20% by weight, in particular from 0.05% to 10% by weight, with respect to the total weight of the composition.

15. Composition according to one of the preceding claims, which is provided in the form of a hair cosmetic composition, such as a hair care lotion, of a shampoo or of a hair conditioner, of a liquid or solid soap for cleaning the scalp, of a product for shaping the hairstyle, of a treatment mask, of a cream or of a foaming gel for cleaning the hair.

16. Method for the cosmetic (non-therapeutic) treatment of human keratinous substances, in particular the hair and/or scalp, in which a cosmetic composition as defined in one of Claims 1 to 15 is applied to said keratinous substances and said keratinous substances are optionally rinsed.

17. Method according to Claim 16, said method being a method for the cosmetic care of the hair and/or scalp, for the purpose of improving their condition and/or their appearance.

18. Non-therapeutic use of a composition as defined in one of Claims 1 to 15 to induce and/or stimulate the growth of human keratinous fibers and/or to slow down their loss and/or to increase their density.
